# EUROPEAN PATENT APPLICATION

(11) **EP 3 594 960 A1**
(43) Date of publication of application: **15.01.2020**
(21) Application number: 19185114.6
(22) Date of filing: 09.07.2019
(51) Int. Cl.: G16H 40/20, G16H 40/63

(54) **DEVICE FOR A NURSE CALL SYSTEM**

(30) Priority: 09.07.2018 US 201816030557
(71) Applicant: Ascom (Sweden) AB, 402 73 Göteborg (SE)
(72) Inventor: UNTERBERGER, Gabriele, 50026 San Casciano in Val di Pesa (IT); VAN RYZIN, Patrick, Bradenton, FL 34208 (US); FOTH, David, Bradenton, FL 34212 (US); CAPPELLETTI, Carlo, 50134 Florence (IT); CHMIELEWSKI, Richard, Bradenton, FL 34203 (US); MEHTA, Sanjay, Sarasota, FL 34233 (US)
(74) Representative: Karl, Christof

(57) **Abstract**

The present invention relates to a device for a nurse call system, wherein the device comprises: a core nurse call functionality; and an embedded viewer adapted to provide access to at least one system external of the nurse call system in order to provide the device with additional functionality. The present invention also relates to a nurse call system and a corresponding method.

## Description

### Field of the invention

The present invention relates to a device for a nurse call system, a nurse call system, and a corresponding method.

### Background of the invention

A nurse call system is a mission critical hospital communication system required in most medical facilities globally and regulated to ensure safe and effective use. The installation and configuration of such nurse call systems typically require dedicated wiring for the support of operational needs which are primarily to annunciate (audibly and visually) the patient's needs using specific nurse call devices. Existing nurse call systems contain numerous devices, for example nurse station devices, in room devices, and peripheral devices and hallway devices. An example of an existing nurse call system is Ascom Telligence.

The mission critical nurse call system must operate reliably 24x7x365. To accomplish this, it is essential to eliminate unknowns to minimize unforeseen impacts to the nurse call system operation, such as limiting access by 3rd party applications. To this end, in the past, nurse call systems have been isolated from other (3rd party) systems to ensure reliability needed to meet regulations. For example, a separate PC/tablet and network have been installed to provide in-room access to 3rd party application, which among other things is a costly solution.

In US2009/0217080, a first plurality of computer devices operable as a nurse call system and having core nurse call functionality residing on an embedded computing platform is provided with additional functionality via software plug-ins that are transmitted to the first plurality of computer devices by a second plurality of computer devices. However, using plug-ins like in US2009/0217080 could lead to regulatory issues, and it may also limit the additional functionality/content available at a given station.

### Summary of the invention

It is an object of the invention to provide an improved device for a nurse call system, which may overcome or at least alleviate one or more of the drawbacks mentioned above.

According to a first aspect of the invention, there is provided a device for a nurse call system, wherein the device comprises: a core nurse call functionality; and an embedded viewer adapted to provide access to at least one system external of the nurse call system in order to provide the device with additional functionality.

The present invention is based on the understanding that an embedded viewer beneficially may be used to provide access to external systems in order to provide the device with additional functionality beyond the core nurse call functionality. In particular, when different external systems are accessed and different contents are retrieved by the embedded viewer, this does not change the computer code on the device (no new code is downloaded, in contrast to a case where a plug-in or applet is downloaded), which in turn means that it is not necessary to seek new regulatory approval as soon as a new additional functionality is provided, which otherwise may be required in some jurisdictions. For example, the present device with the embedded viewer may comply with the UL standard for Safety for Hospital Signaling and Nurse Call Equipment, UL 1069. Furthermore, the embedded viewer may provide access to generally any external system (which for example could allow access to basically any web page or web application), meaning that many different additional functionalities readily can be added to the present device, again without recertification for compliance with UL 1069.

The 'core nurse call functionality' may include but is not limited to creating alerts and events, viewing alerts, creating and managing workflows, establishing audio communication with another device, and overhead paging, as realized by means of various components including software and/or hardware of the device. The 'additional functionality' may include but is not limited to service task list, rounding tasks, patient status, bed management, patient entertainment and education, charting, and access to health information. Furthermore, 'embedded' indicates that the viewer is for a device typically not thought of as a (personal) computer, and/or that the viewer is specialized for the particular device that it runs on. The present device may be a nurse station device or an in-room device, for example.

Various measures to prevent the embedded viewer from compromising the core nurse call functionality may be implemented. For example, the core nurse call functionality may have precedence over the embedded viewer, e.g. with respect to the processor, memory, etc. of the device. Furthermore, the embedded viewer may be restricted with respect to how much capacity of the device (e.g. processing power, memory space, etc.) that the embedded viewer can use. Furthermore, the embedded viewer may in operation run in a restricted environment, like a so-called sandbox. Furthermore, the embedded viewer may in operation be monitored through application performance management, which is known per se.

The device may further comprise a display configured to interchangeably show a user interface of the core nurse call functionality and content retrieved by the embedded viewer. The display may be a touch display.

The display may further be configured to show a bar comprising navigational touch points allowing a user to switch between the user interface of the core nurse call functionality and the content retrieved by the embedded viewer. This allows the user to seamlessly switch between the core nurse call functionality and the additional functionality. The bar may be shown at all times, so that when the content retrieved by the embedded viewer is shown, the user can readily go back to the user interface of the core nurse call functionality. The navigational touch points may be icons or tabs, for example.

A navigational touch point of said navigational touch points, which navigational touch point is associated with content retrieved by the embedded viewer, may include a badge indicating information related to at least one of the content retrieved by the embedded viewer and the access to the at least one system external of the nurse call system. In this way, a staff member can easily get basic information pertaining to a particular additional functionality. The information related to the content retrieved by the embedded viewer may for example be a numerical value indicating a room or a number of actions to be completed. The information related to the access to the external system(s) may for example be a symbol indicating that the connection to the external system(s) is lost.

Content retrieved by the embedded viewer from the at least one system external of the nurse call system may be customized based on information from the device. The information from the device may include information about at least one of the location of the device (e.g. room number), the staff member using the device (e.g. authorization level), and the patient currently in this location (e.g. patient name and/or ID). Content retrieved by the embedded viewer may hence be customized so as to be more contextually aware, which in turn may lessen the burden for the user of the device.

The embedded viewer may be adapted to retrieve and present HyperText Markup Language, HTML, based content from the at least one system external of the nurse call system. In this way, the device may retrieve content from any (third partly) external system that supports HTML without requiring dedicated applications to be purpose built for the device.

The embedded viewer may be adapted to retrieve content from the at least one system external of the nurse call system via the Hypertext Transfer Protocol, HTTP.

The embedded viewer may be a custom widget. That is, the embedded viewer may be a relatively simple and easy-to-use software application that is specially developed for the device provider. In contrast to a regular web browser, the present embedded viewer may for example lack address bars, navigation buttons, and the like.

The present device may be a staff operated device, for example a nurse station device, like Ascom's Staff Console or Annunciator but with the embedded viewer, or an in-room device, like Ascom's Staff Station or Workflow Station but with the embedded viewer, or a corridor device.

According to a second aspect of the invention, there is provided a nurse call system comprising at least one device with a nurse call functionality and an embedded viewer adapted to provide access to at least one system external of the nurse call system. This aspect may exhibit the same or similar features and technical effects as the first aspect, and vice versa.

The at least one system external of the nurse call system may be at least one third-party system, i.e. an entity independent of the nurse call system (provider).

The nurse call system may further comprise an application programming interface, API, adapted to allow reception of data from the at least one system external of the nurse call system in order to annunciate an event using an annunciating capability of the at least one device based on said data. In this way, the infrastructure already in place (i.e. the nurse call system) may be leveraged as its annunciating capabilities can be used also by external systems. That is, utilization of the nurse call system may be improved. The application programming interface here generally functions as a software intermediary that allows the nurse call system and the external system(s) to share information and data with each other. Furthermore, the API may provide for an authenticated and encrypted channel of receiving the event via well-defined interfaces.

It should be noted that the application programming interface feature may be used also with devices of the nurse call system not having the embedded viewer. Hence it is envisaged a nurse call system, comprising: at least one device with an annunciating capability; and an application programming interface, API, adapted to allow reception of data from at least one system external of the nurse call system in order to annunciate an event using said annunciating capability based on said data.

The annunciating capability of the at least one device may be selected from the group comprising visual notification means, audible notification means, a display, and combinations thereof. Visual notification means may for example be the LEDs of a dome light corridor device. Audible notification means may for example be the speaker of an in-room speech device or the speaker of a nurse station device. The display may for example be the display of a nurse station device, or the display of a corridor display device. An external system may for example use the display of a nurse station device to indicate status of patients, such as fall risk. In another example, an external system may use the LEDs of a dome light corridor device to request an action to be performed in a specific location, such as rounding on patients. In another example, an external system may use the speaker of a nurse station device to annunciate that a patient that is a "fall risk" has gotten out of bed by himself with an audio tone.

The application programming interface preferably allows for digital bi-directional communication between the nurse call system and the at least one system external of the nurse call system. In this way, data can be sent not only form the external system(s) to the nurse call system, but also from the nurse call system to the external system(s). The latter may for example include sending nurse call event to an external system for the purpose of indicating an update to patient status e.g. allergy conditions on a patient, or for the purpose of storing something into a database for future reporting and auditing purpose.

According to a third aspect of the invention, there is provided a method, wherein a device of a nurse call system comprises a core nurse call functionality and an embedded viewer, the method comprising: accessing at least one system external of the nurse call system by means of the embedded viewer in order to provide the device with additional functionality. This aspect may exhibit the same or similar features and technical effects as the first aspect and/or the second aspect, and vice versa.

The method may further comprise: receiving data from the at least one system external of the nurse call system via an application programming interface, API, of the nurse call system; and annunciating an event using an annunciating capability of said device based on the received data.

The steps in the previous paragraph may be performed also if the device does not have the embedded viewer. Hence it is envisaged a method, which method comprises: receiving data from at least one system external of a nurse call system via an application programming interface, API, of the nurse call system; and annunciating an event using an annunciating capability of a device of the nurse call system based on the received data.

### Brief description of the drawings

These and other aspects of the present invention will now be described in more detail, with reference to the appended drawings showing one or more embodiments of the invention.
Fig. 1 schematically illustrates a device for a nurse call system according to an embodiment of the invention.
Fig. 2 schematically illustrates a nurse call system according to one or more embodiments of the invention.
Fig. 3 is a flow chart of a method according to an embodiment of the invention.
Figs. 4a-b show exemplary displays of the device of fig. 1.
Fig. 5 is a flow chart of another method according to the present disclosure.

### Detailed description of the invention

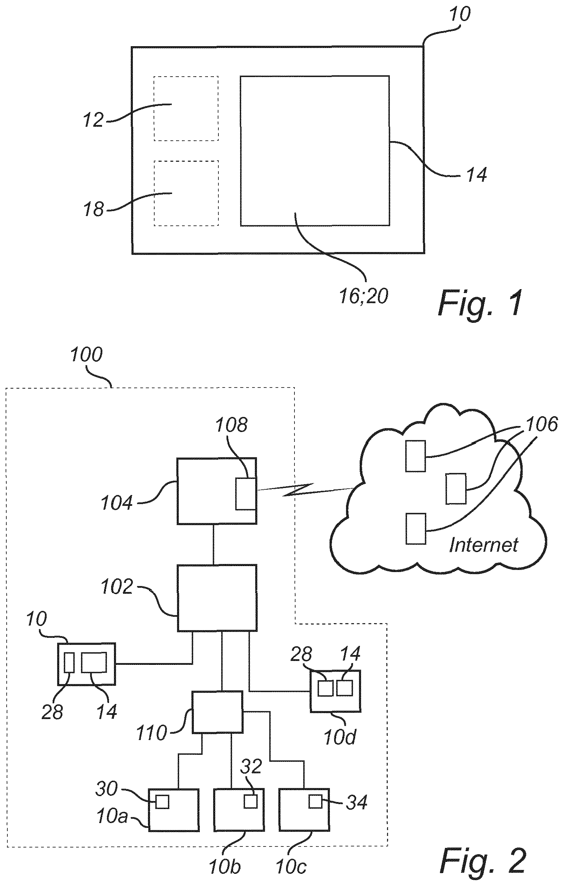

Fig. 1 schematically illustrates a device 10 for a nurse call system 100 according to an embodiment of the invention. The device 10 may also be referred to as a nurse call system device 10.

The device 10 comprises a core nurse call functionality 12. The core nurse call functionality 12 may include but is not limited to creating alerts and events, viewing alerts, creating and managing workflows, establishing audio communication with another device, and overhead paging, as realized by means of various components including software and/or hardware, in particular a display 14, of the device 10. The display 14 may be configured to show a local user interface 16 of the core nurse call functionality 12. The user interface 16 may include a nurse call screen, an alert list, a dial interface, etc. The display 14 may be a touch display. The device 10 may be a staff operated device, for a nurse station device located at a nurse station.

The device 10 may be connected to the remaining nurse call system 100, as seen in fig. 2. In particular, the device 10 may be connected to a router 102, which provides connectivity (e.g. Ethernet; data link layer) to the device 10. The router 102 in turn may be connected to a bridge PC 104. The nurse call system 100 may use IP as network layer protocol.

The device 10 further comprises an embedded viewer 18. The embedded viewer 18 is software, namely a software application, stored on the device 10. The embedded viewer 18 may for example be a custom widget, namely a specially developed web browser without address bars, navigation buttons, and the like. The embedded viewer 18 is adapted to provide access to at least one system 106 (see fig. 2) external of the nurse call system 100, in order to provide the device 10 with additional functionality. The additional functionality may include but is not limited to service task list, rounding tasks, patient status, bed management, patient entertainment and education, charting, and access to health information.

Specifically, the embedded viewer 18 may be adapted to retrieve HTML based content 20 from the at least one system 106 external of the nurse call system 100 system via HTTP (application layer), and to present or show the retrieved content 20 on the display 14. The embedded viewer 18 may for example be adapted to present content from a web application running on the at least one system 106. The at least one system 106 may be one or more remote servers (e.g. web servers), and the at least one system 106 may be accessed by means of the embedded viewer 18 of the device 10 via the aforementioned router 102 and bridge PC 104. The bridge PC 104 may be connected to the internet. Furthermore, the at least one system 106 may be third-party systems, i.e. entities independent of the provider of the nurse call system 100.

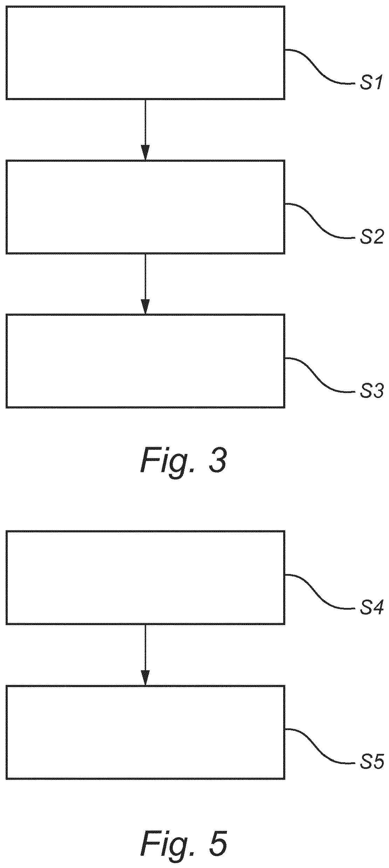

In use, and with further reference to fig. 3, the at least one system 106 external of the nurse call system 100 is accessed by means of the embedded viewer 18 (step S1), in order to provide the device 10 with the additional functionality. The embedded viewer 18 may retrieve content 20 from the accessed at least one system 106 (step S2), and present or show the content 20 on the display 14 (step S3). Notably, no code or plugins or applets or applications are downloaded to the device 10 when different systems 106 are accessed and different contents 20 are retrieved and presented by the embedded viewer 16. In this way, no recertification of the device 10 in accordance with UL 1069 is required when adding (new) additional functionality to the device 10.

Various measures to prevent the embedded viewer 18 from compromising the core nurse call functionality 12 may be implemented in the device 10. For example, the core nurse call functionality 12 may have precedence over the embedded viewer 18, e.g. with respect to the processor, memory, etc. of the device 10. Furthermore, the embedded viewer 18 may be restricted with respect to how much capacity of the device 10 (e.g. processing power, memory space, etc.) that the embedded viewer 18 can use. Furthermore, the embedded viewer 18 may in operation run in a restricted environment, like a so-called sandbox. Furthermore, the embedded viewer 18 may in operation be monitored through application performance management.

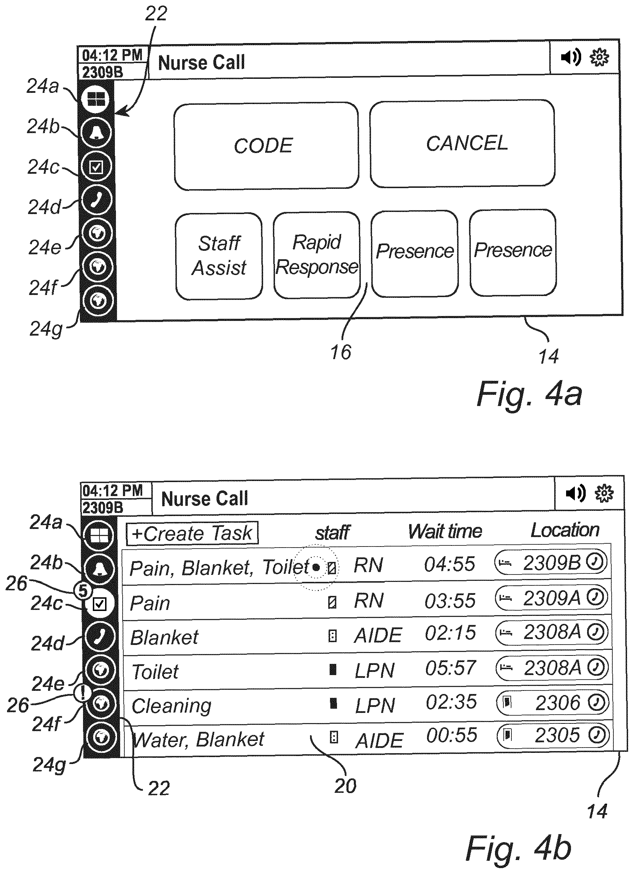

With further reference to figs. 4a-b, the display 14 of the device 10 may interchangeably show the user interface 16 of the core nurse call functionality 12 and the content 20 retrieved by the embedded viewer 18. To this end, the display 14 may further be configured to show a bar 22 comprising navigational touch points 24a-f allowing a user of the device 10, for example a staff member, to seamlessly switch between the user interface 16 and the content 20 retrieved by the embedded viewer 18. The bar 22 is in figs. 4a-b vertical and positioned along the left edge of the display 14, but it could have other orientations and/or positions. The navigational touch points 24a-g may for example be icons, as in figs. 4a-b. Each navigational touch point 24a-g may be associated with the user interface 16 of the core nurse call functionality 12 or content 20 retrieved by the embedded viewer 18. Here, navigational touch point 24a is associated with a nurse call screen of the user interface 16, navigational touch point 24b is associated with an alert list of the user interface 16, navigational touch point 24c is associated with a service task list retrieved by the embedded viewer 18, navigational touch point 24d is associated with a dial interface of the user interface 16, and navigational touch point 24e-g are associated with various other additional functionalities as realized by content retrieved by the embedded viewer 18. The bar 22 may be shown at all times, so that when the content 20 retrieved by the embedded viewer 18 is shown as in fig. 4b, the user can readily go back to the user interface 16 of the core nurse call functionality 12, for example by clicking on navigational touch point 24a. Likewise, when the user interface 16 of the core nurse call functionality 12 is shown on the display 14, for example the nurse call screen as illustrated in fig. 4a, the user can readily go to content 20 retrieved by the embedded viewer 18, for example by clicking on for example navigational touch points 24c. The user interface 16 of the core nurse call functionality 12 and content 20 retrieved by the embedded viewer 18 is interchangeably shown in the same area of the display 14, and the user may not know if he/she views content 20 retrieved by the embedded viewer 18 rather than the user interface 16 of the core nurse call functionality 12.

Navigational touch points associated with (switching to) content 20 retrieved by the embedded viewer 18, for example navigational touch points 24c and 24e, may include a badge 26 indicating information related to at least one of the content 20 retrieved by the embedded viewer 10 and the access to the at least one system 106 external of the nurse call system 102. The information related to the content 20 retrieved by the embedded viewer 18 may for example be a numerical value indicating a room or a number of actions to be completed (the badge of navigational touch points 24c), and the information related to the access to the system(s) 106 may for example be a symbol indicating that the connection to the external system(s) 106 is lost (the badge of navigational touch points 24f).

Furthermore, content 20 retrieved by the embedded viewer 18 from the at least one system 106 external of the nurse call system 100 may be customized based on information from the device 10. The information from the device 10 may include information about at least one of the location of the device 10 (e.g. room number), the staff member using the device 10 (e.g. authorization level), and the patient currently in this location (e.g. patient name and/or ID). This information may be sent from the device 10 when the at least one system 106 is accessed. The information may for example be included in a URL request. If the information for example includes patient name and/or ID, the system 106 may know that this patient has dietary restriction, whereby the content 20 retrieved by the embedded viewer 18 from the system 106 does not include the option to order food.

Returning to fig. 2, the nurse call system 100 may further comprise an application programming interface (API) 108. The API 108 may for example reside on the bridge PC 104. The API 108 is adapted to allow reception of data from at least one system external of the nurse call system 100, like system(s) 106, in order to annunciate an event using an annunciating capability of the device 10 based on said data. The event may be referred to as an external event, in that it may be initiated or created by the at least one system 106 external of the nurse call system 100. The external event should be contrasted to internal events created inside the nurse call system 100 (i.e. core nurse call functionalities), such as a patient pressing a button on a physical in-room device of the nurse call system 100 to create a request or notification for assistance. The annunciating capability of the device 10 may for example be the aforementioned display 14, but it could also be a speaker 28 (audible notification means) of the device 10.

The core nurse call functionality of the nurse call system 100 is preferably totally separate from the external events based on data received via the API 108, and hence cannot compromise the core nurse call functionality of the nurse call system 100. That is, the reception of data from the external system(s) 106 is through the (programically available software) API 108 and not through physical devices as explained above for internal events. The external event may be created and owned by the external system 106, and the API 108 basically allows a software application on for example the device 10 to receive data representing the external event and e.g. show the external event on the display 14.

Furthermore, internal and external events can be harmonized so to annunciate all events with relevant prioritization and visual/audible indications. For example, both internal and external events may be shown in the same alert list shown on the display 14 of the device 10, wherein critical events may be shown on top of the alert list. Different iconography or colors could be used in the alert list to distinguish between internal and external events. In another example, internal and external events can be distinguished by different lights on a dome light corridor device (see below).

The API 108 also works with devices of the nurse call system 100, which devices do not include the embedded viewers. Such (nurse call system) devices may include, but are not limited to, a dome light corridor device 10a, an in-room speech device 10b, a corridor display device 10c, and nurse station device 10d without the embedded viewer18. The dome light corridor device 10a, the in-room speech device 10b, and the corridor display device 10c may be connected to the router 102, preferably via a gateway 110. The nurse station device 10d without the embedded viewer18 may be connected directly to the router 102. The dome light corridor device 10a may have an annunciating capability in the form of LEDs 30 (visual notification means). The in-room speech device 10b may have an annunciating capability in the form of a speaker 32 (audible notification means). The corridor display device 10c may have an annunciating capability in the form of a display 34. The nurse station device 10d may have annunciating capability in the form of a display 14 and/or a speaker 28, for example.

The API 108 functions as a software intermediary that allows the nurse call system 100 (including the devices 10 and/or 10a-d) and the at least one system 106 external of the nurse call system 100 to share information/data with each other. Preferably, the API 108 allows for digital and/or bi-directional communication between the nurse call system 100 and the at least one system 106 external of the nurse call system 100.

In use, and with further reference to fig. 5, data is received data from the at least one system 106 external of the nurse call system 100 via the API 108 (step S4), and an external event is annunciated using the annunciating capability of at least one of the devices 10 and 10a-d based on the received data (step S5).

An external system 106 may for example use the display 16 of device 10 or 10d to indicate status of patients, such as fall risk. In another example, another external system 106 may use the LEDs 30 of the dome light corridor device 10a to request an action to be performed in the specific location associated with that dome light corridor device 10a, such as rounding on patients. In another example, an external system 106 may use the speaker 32 of the in-room speech device 10b to audibly annunciate a bed exit event or an equipment alert from a device in the room or elsewhere. In another example, an external system 106 may use the display 34 of the corridor display device 10c to indicate event alert status to staff members in the corridor.

The automated system of the present application may be just hardware, but is generally implemented in software in a hardware system comprising a data processor, associated memory and input output devices (e.g., keyboard, mouse, displays, printers, microphone, speakers, etc.). The processor routines and data may be stored on a non-transitory computer readable medium as a computer program product, which may be read and executed by one or more processors. The system may, for example, be a standalone computer, a network of devices (e.g., client and server devices), a mobile device or combination thereof.

The person skilled in the art realizes that the present invention by no means is limited to the preferred embodiments described above. On the contrary, many modifications and variations are possible within the scope of the appended claims.

## Claims

1. A device for a nurse call system, wherein the device comprises:
a core nurse call functionality; and
an embedded viewer adapted to provide access to at least one system external of the nurse call system in order to provide the device with additional functionality.

2. A device according to claim 1, wherein the core nurse call functionality has precedence over the embedded viewer.

3. A device according to claim 1, wherein the embedded viewer is restricted with respect to how much capacity of the device that the embedded viewer can use.

4. A device according to claim 1, wherein the embedded viewer in operation runs in a restricted environment.

5. A device according to claim 1, wherein the embedded viewer in operation is monitored through application performance management.

6. A device according to claim 1,
wherein the device further comprises a display configured to interchangeably show a user interface of the core nurse call functionality and content retrieved by the embedded viewer, and
wherein the display is further configured to show a bar comprising navigational touch points allowing a user to switch between the user interface of the core nurse call functionality and the content retrieved by the embedded viewer, and
wherein a navigational touch point of said navigational touch points, which navigational touch point is associated with content retrieved by the embedded viewer, includes a badge indicating information related to at least one of the content retrieved by the embedded viewer and the access to the at least one system external of the nurse call system.

7. A device according to claim 1, wherein content retrieved by the embedded viewer from the at least one system external of the nurse call system is customized based on information from the device.

8. A device according to claim 1, wherein the embedded viewer is adapted to retrieve and present HyperText Markup Language, HTML, based content from the at least one system external of the nurse call system.

9. A device according to claim 1, wherein the embedded viewer is adapted to retrieve content from the at least one system external of the nurse call system via the Hypertext Transfer Protocol, HTTP.

10. A device according to claim 1, wherein the embedded viewer is a custom widget.

11. A device according to claim 1, wherein the device is a staff operated device.

12. A nurse call system comprising at least one device with a nurse call functionality and an embedded viewer adapted to provide access to at least one system external of the nurse call system, and
wherein the at least one system external of the nurse call system is at least one third-party system.

13. A nurse call system according to claim 12, further comprising an application programming interface, API, adapted to allow reception of data from the at least one system external of the nurse call system in order to annunciate an event using an annunciating capability of the at least one device based on said data.

14. A nurse call system according to claim 13, wherein the annunciating capability of the at least one device is selected from the group comprising visual notification means, audible notification means, a display, and combinations thereof.

15. A nurse call system according to claim 13, wherein the application programming interface allows for digital bi-directional communication between the nurse call system and the at least one system external of the nurse call system.
